# EUROPEAN PATENT SPECIFICATION

(11) **EP 2 442 828 B1**
(45) Date of publication and mention of the grant of the patent: **03.08.2016**
(21) Application number: 10725740.4
(22) Date of filing: 18.06.2010
(51) Int. Cl.: A61K 39/35, A61K 39/36, A61K 39/39, A61K 9/00, A61P 37/08, A61K 38/00, A61K 39/00

(54) **NOVEL MANNER OF ADMINISTRATING ALLERGEN IN ALLERGEN SPECIFIC IMMUNOTHERAPY**
NEUARTIGE WEISE ZUR VERABREICHUNG VON ALLERGEN BEI EINER ALLERGENSPEZIFISCHEN IMMUNTHERAPIE
Nouveau procédé d'administration d'allergène dans une immunothérapie spécifique allergénique

(30) Priority: 19.06.2009 EP 09163261
(43) Date of publication of application: 25.04.2012
(73) Proprietor: ALK-ABELLÓ S.A., 28037 Madrid (ES); Domínguez, Joaquín Sastre, 28760 Madrid (ES)
(72) Inventor: DOMÍNGUEZ, Joaquín Sastre, E-28760 Madrid (ES); NIETO, Pilar Rico, E-28002 Madrid (ES)
(74) Representative: Inspicos P/S
(86) International application number: PCT/EP2010/058663
(87) International publication number: WO 2010/146171

(56) References cited:
- NAGAI TAKASHI ET AL: "Immediate-type human insulin allergy successfully treated by continuous subcutaneous insulin infusion" INTERNAL MEDICINE (TOKYO), vol. 36, no. 8, August 1997 (1997-08), pages 575-578, XP002554631 ISSN: 0918-2918
- CASTERA V ET AL: "Systemic allergy to human insulin and its rapid and long acting analogs: successful treatment by continuous subcutaneous insulin lispro infusion" DIABETES & METABOLISM, vol. 31, no. 4, Part 1, September 2005 (2005-09), pages 391-400, XP002554632 ISSN: 1262-3636
- MOYES V ET AL: "Insulin allergy in a patient with type 2 diabetes successfully treated with continuous subcutaneous insulin infusion" CLINICAL AND EXPERIMENTAL ALLERGY, BLACKWELL SCIENTIFIC PUBLICATIONS, LONDON, GB, vol. 35, no. 10, 1 October 2005 (2005-10-01), page 1417, XP009125449 ISSN: 0954-7894 [retrieved on 2005-10-20]
- RADERMECKER R P ET AL: "Allergy reactions to insulin: effects of continuous subcutaneous insulin infusion and insulin analogues" DIABETES-METABOLISM RESEARCH AND REVIEWS, vol. 23, no. 5, July 2007 (2007-07), pages 348-355, XP002554633 ISSN: 1520-7552
- PATRIARCA G ET AL: "SUBLINGUAL DESENSITIZATION IN PATIENTS WITH WASP VENOM ALLERGY: PRELIMINARY RESULTS" INTERNATIONAL JOURNAL OF IMMUNOPATHOLOGY AND PHARMACOLOGY, vol. 21, no. 3, July 2008 (2008-07), pages 669-677, XP009125515 ISSN: 0394-6320
- HILDEBRANDT P ET AL: "Basal rate subcutaneous insulin infusion: absorption kinetics and relation to local blood flow." DIABETIC MEDICINE : A JOURNAL OF THE BRITISH DIABETIC ASSOCIATION 1988 JUL-AUG LNKD- PUBMED:2970915, vol. 5, no. 5, July 1988 (1988-07), pages 434-440, XP002593594 ISSN: 0742-3071
- LARENAS-LINNEMANN DÉSIRÉE: "Subcutaneous and sublingual immunotherapy in children: complete update on controversies, dosing, and efficacy" CURRENT ALLERGY AND ASTHMA REPORTS, CURRENT SCIENCE, US, vol. 8, no. 6, 1 November 2008 (2008-11-01), pages 465-474, XP009112413 ISSN: 1529-7322
- ROSSI ET AL: "Evaluation of serum IgG4 antibodies specific to grass pollen allergen components in the follow up of allergic patients undergoing subcutaneous and sublingual immunotherapy" VACCINE, BUTTERWORTH SCIENTIFIC. GUILDFORD, GB, vol. 25, no. 5, 8 December 2006 (2006-12-08), pages 957-964, XP005798887 ISSN: 0264-410X
- DATABASE MEDLINE [Online] US NATIONAL LIBRARY OF MEDICINE (NLM), BETHESDA, MD, US November 2011 SASTRE JOAQUÍN ET AL: 'Rush allergen subcutaneous immunotherapy administered with infusion pump.' Database accession no. NLM22018619 & SASTRE JOAQUÍN ET AL: "Rush allergen subcutaneous immunotherapy administered with infusion pump.", ANNALS OF ALLERGY, ASTHMA & IMMUNOLOGY : OFFICIAL PUBLICATION OF THE AMERICAN COLLEGE OF ALLERGY, ASTHMA, & IMMUNOLOGY NOV 2011, vol. 107, no. 5, November 2011 (2011-11), pages 459-460, ISSN: 1534-4436

## Description

### Technical Field

The present Invention belongs to the technical field of desensitizing an allergic individual toward an allergen causing an IgE-mediated allergic immune response in the individual.

### Background

Allergen Specific Immunotherapy (SIT), also known as Specific Allergy Vaccination (SAV), is a vaccine program used In the treatment of individuals suffering from an IgE-mediated allergic disease. By this vaccine It Is aim to permanently reduce the individual's sensitivity to the allergen causing the allergic disease.

Different routes of administration may in principle be applled for the administration of the allergen to the individual, but so far only the subcutaneous injection route and the sublingual administration route have been proven both clinical safe and clinical effective.

Subcutaneous allergen specific Immunotherapy (SCIT) is considered sufficiently safe, but the potential for an adverse reaction Is always present. There are several types of adverse reactions that occur with SCIT, both local and systemic. Although these reactions are rare, they can be life-threatening. Local reactions occur at the site of injection and they can be divided Into reactions that occur before (immediate reactions) and later than 30 min after an injection (delayed reactions). They are usually IgE-mediated and delayed reactions may start several hours later with local edema, erythema (redness), itching and pain. Larger local reactions may be associated with IgG complex (Arthrus) reaction, manifested by pain, tenderness, and hard swelling. Systemic reactions have a low Incidence and manifestations can Include: urticaria, angloedema, increased respiratory symptoms (nasal or pulmonary), increased ocular symptoms, and hypotension.

Because of those safety concerns with subcutaneous administration of allergen, the vaccine program is initiated by an up-dosing phase, where increasing doses of the allergen are administered with intervals of usually 3 to 7 days between administrations until the treatment dose (maintenance dose) is reached, which Is regarded as the effective and tolerable dose in ameliorating the symptoms associated with subsequent exposure to the causative allergen (Bousquet *et al.* 1998). At each subcutaneous administration, it is required that the patient is under observance of medical educated personnel due to risk of getting adverse reactions to the treatment. Typically, the Injections in the up-dosing phase (build-up phase) are given as single Injections on a weekly or bi-weekly basis over a period of 3 to 6 months, typically about 4 months. Once the therapeutically effective dose (maintenance dose) has been reached, the allergen vaccine can be administered less frequently, such as monthly, every six weeks or eventually on a bi-monthly basis. The monthly to bi-monthly vaccination is usually continued for 3 to 5 yrs. This process reduces the allergic response that is otherwise triggered by allergen exposure.

Despite the clear benefits of SIT, it is not a widely used treatment principle for fighting IgE-mediated allergic disease in that only a small percentage of allergic patients subscribe to this treatment. Inconvenience is one of the primary reasons for discontinuation of SIT. In particular, the adherence to subcutaneous administered allergen in SIT treatment is problematic due to time constraints, adverse reactions and inconvenience.

Strategies to improve the safety, efficacy and compliance of SCIT continue to be the focus of several studies. For example, efforts have focused on the incorporation of more rapid up-dosing (build-up) phases so as to reach the maintenance dose by use of fewer injections and thus in a shorter time, such as by the application of either rush or cluster schedules (Cox L. 2008). With rush dosing schedules, multiple injections are given daily or with few days interruption, where a maintenance dose is reached after 1 or several days. However, there are disadvantages of accelerated immunotherapy schedules due to potentially higher risk of systemic reactions and the need for increased staff time and resources in the medical facility (Cox L. 2008) and a maintenance dose as high as in conventional SCIT is not achieved. Cluster dosing consists of 2 or more injections per day, but visits are typically 1 or 2 days per week. Maintenance dose with cluster dosing is usually not achieved as rapidly as with rush, but in comparison with conventional schedules, there does not appear to be an increased incidence of systemic reactions.

Accelerated immunotherapy schedules offer the advantage of achieving clinical benefits of SIT sooner, while requiring less patient time because of a reduced number of visits at a medical facility during the up-dosing phase. Changes in objective parameters associated with effective immunotherapy are also seen earlier with accelerated schedules. The disadvantage of accelerated immunotherapy schedules is that they can be associated with greater risk, particularly with aeroallergens where accelerated immunotherapy often is associated with increased incidence of adverse reactions.

Another approach in diminishing adverse reactions associated with SCIT is the use of modified allergens, such as glutaraldehyde-modified allergen extracts. These vaccines maintain or increase IgG antibody recognition, while significantly reducing IgE-reactivity, which allows for shortening of the build-up phase as well as for the administration of higher maintenance doses than with non-modified allergens (Casanovas M *et al.* 2006). Modified allergens can be given as four pre-seasonal injections with significant reduction of symptoms and in the requirement for medication.

However, it is still highly desirable to reduce adverse reactions and increase patient compliance by reducing the number of visits at a medical facility during the up-dosing phase and preferably also during the maintenance phase, while maintaining the established clinical relevant efficacy of SCIT.

### Summary of Invention

The present inventors have surprisingly found it possible to significantly reduce the number of doses in the up-dosing phase of SCIT, while still achieving a good safety profile in comparison to conventional SCIT treatment, provided the allergy vaccine is delivered, preferably continuously, by a significantly slower flow rate to the subcutaneous tissue of an individual in need thereof and for a longer period than usually practiced with subcutaneous bolus injections. The slow delivery of the allergen may advantageously be accomplished by means of an infusion pump.

Without being limited to a particular theory, the inventors emphasise that the slow distribution of the allergy vaccine in subcutaneous tissue prevents strong inflammasome complex activation and confers sustained release of the allergen to the tissue, which in turn may induce tolerance responses to the allergen.

It should be considered, that whenever an allergen composition is administered, the first doses are known to induce a strong Th2-cell mediated allergic response, while after the onset of regulatory responses, there is a shift to Th1-cell responses as SCIT is continued for a prolonged period. Strong activation of inflammasome in the first doses will thus represent an increased risk for the appearance of adverse reactions (Th2-cell activation). It is believed that by administering the allergen composition by low flow rate for a longer period than normally applied with subcutaneous injection (bolus injection) the activation of the inflammasome can be minimized. Without being limited to any particular theory, it is believed that the allergen composition slowly diffuses into the surrounding tissues, thereby reducing tissue damage and minimising inflammasome activation and creating an allergen reservoir that by continuous stimulation of the immune system will induce a Th3-cell response. A Th3-cell mediated immune response is regarded as the desirable type of immune response in SCIT with the aim of achieving clinical relevant reduction in the allergy symptoms (Nouri-Aria KT *et al* 2008). After the leap to a Th1-cell profile is established, conventional immunotherapy, such as by direct subcutaneous injection, can be applied without the risk of adverse reactions.

Besides, the inventors have also found that the switch from administering the allergy vaccine by infusion pump administration to subcutaneous bolus injection is possible without any safety concern. That is to say that the present inventors have recognised an improved manner of conducting SCIT that includes subcutaneous infusion administration of the allergen composition in the up-dosing phase, whereas the allergen composition may still be administered by the current manner, subcutaneous bolus injections.

For example, it has been possible to reduce the number of administrations in the up-dosing phase from about 7 to 15 administrations in total to about 2 to 4 administrations in total by use of the novel manner of administering the allergen composition, thus resulting in significant shorter duration of the up-dosing phase allowing for the up-dosing phase to be completed within 2 to 4 weeks of time.

The number of administrations may to some extent depend on the particular formulation of the allergen composition as well as its content of allergens. However, it was shown possible to reduce the number of administrations to only 2-3 for all the various allergen compositions tested, thus indicating that this novel concept of SCIT shall be acknowledged as a general concept for reducing the number of administrations in the up-dosing phases for all types of allergy vaccines usually applied in SCIT.

Infusion pumps have been widely used for subcutaneous administration of different drugs and for several years. The portable syringe pump was invented by Wright in 1979 to deliver desferrioxamine to patients with thalassemia. In the same year Russell suggested its use for infusions in terminal malignant disease. The continuous subcutaneous infusion of drugs by a small portable pump (sometimes called a "syringe driver") is a major advance in terminal care, particularly for symptom control even in the home. It has been used in alleviating immunodeficiency by IgG replacement therapy (Gardulf A. *et al,* 2001). Advantageously, the patient can remain fully ambulant and the pump is small, portable and battery-operated.

Continuous subcutaneous insulin infusion therapy has also been used in the treatment of cutaneous insulin allergy, where itchy skin wheals develop at the insulin injection site as an immunological response to the injected insulin (Nagai *et al,* 1997). Some patients in addition to the local allergic response also develop systemic reactions, such as anaphylactic shock, following insulin bolus injections. For example, continuous subcutaneous insulin infusion therapy was applied for the treatment of a diabetic patient with cutaneous as well as systemic symptoms on an allergic immune response after bolus injection with insulin by using an initial basal rate with slowly increasing the dose of insulin, such as 0.01 U/h the first day, 0.02 U/h the second day, 0.05 U/h the third day, then doubling the dose every day until 0.8 U/h after 7 days, and finally 1 U/h the eight day and 1.3 U/h the ninth day (Castera *et al,* 2005). As concluded by the authors, real desensitisation as indicated by negative skin test to the sensitising insulin and reduction in specific IgE antibodies to the sensitising insulin was not shown, but the effect was rather due to tolerance induction (Castera *et al,* 2005). Furthermore, another diabetic patient with allergic symptoms following insulin therapy was treated with insulin using continuous subcutaneous infusion through a pump building up gradually to the required dosed of treating diabetes. It was concluded that this treatment reduced the local and systemic symptoms on insulin induced allergy, but that the symptoms suggest a type III hypersensitivity, which does not respond to desensitization (Moyes V *et al,* 2005). Thus, the principle of using continuous subcutaneous infusion has been used in the administration of insulin to diabetic patients exhibiting an allergic reaction towards subcutaneous injected insulin with the aim of administering the required dose for treating their diabetes and at the same time inducing a better tolerance to insulin.

Neither in the field of allergen specific immunotherapy nor in the field of vaccination therapy there is reported any previous experience with this form of administering an allergen composition.

Advantageously, the new manner of administering the allergen composition in SCIT has the effect that in comparison to conventional SCIT it is possible to;
- Increase patient compliance in that the number of doses/administrations of single doses required to reach the maintenance phase is reduced, which in turn allows for significant reduction in the number of patient's visit to a medical facility;
- Increase the safety profile of SCIT in that the number of immediate and delayed responses associated with the allergen immunotherapy is reduced;
- Reduce errors in SCIT administration in that fewer vials with different doses of allergy vaccine is needed, such as just one vial or two vials with different concentration of allergen instead of a whole range of vials with increasing doses of allergen composition, thus lowering the risk of the medical personnel to apply the wrong vial.

Accordingly, a first aspect of the present invention relates to an allergen composition comprising an inhalation allergen or a venom allergen associated with IgE-mediated allergy, formulated to be suitable for subcutaneous administration, for use in subcutaneous specific allergen immunotherapy, which comprises an up-dosing phase and a maintenance phase, and a dose of the allergen composition in the up-dosing phase is administered to an individual in need thereof by use of subcutaneous infusion using a continuous flow at a flow rate not exceeding 0.05 ml/min within a period in the range of 5 to 180 minutes and wherein the up-dosing has a duration of 2-6 weeks and the number of administrations of single doses in the up-dosing phase is from 2-4.

The flow rate not exceeding 0.05 ml/min may be provided by an infusion pump operated at a flow rate not exceeding 0.05 ml/min.

### Detailed Description

### The following definitions are used herein:

The term "allergen composition" is intended to define any composition containing one or more single allergens, including allergens isolated from a biological allergen source or allergens recombinantly produced as well as hypo-allergenic variants thereof. The allergen composition may be presented in a form for "ready-to-use" or may require dissolving or dilution before being administered.

The term "allergy vaccine" refers to a final allergen composition ready for being administered to an individual in need thereof.

The phrase, "subcutaneous allergen specific immunotherapy (in short; SCIT)" refers to the desensitization of an allergic individual having an IgE-mediated allergic disease, wherein one or more of the allergen(s) to which the individual is sensitized to is administered repeatedly as single bolus injections by the subcutaneous route during a course of several months/years with the aim of achieving clinical relevant reduction in the allergic symptom score. Usually, the treatment is initiated with an up-dosing phase followed by a maintenance phase.

The term "up-dosing phase" means a period of treatment during which the doses of the allergen composition are administered in gradually increasing doses to reach a full dose level, which is then used in the following maintenance phase. The up-dosing phase ends when the said full dose level is reached, i.e. immediately subsequent to the administration of the first full dose.

The term "maintenance phase" means a period of treatment in continuation of the up-dosing phase and during which a full dose of allergen composition is administered, the maintenance phase starting immediately subsequent to the administration of the first full dose.

The term "treating or treatment" means partly of wholly curing, alleviating symptoms or inhibiting causes of symptoms.

The term "IgE-mediated allergic disease" designates the type of allergy characterized by the presence of allergen-specific antibody of the IgE class in the circulation and on the surface of mast-cells and basophils. Typically, a pronounced inflammation of the mucosa of the target organ is usually seen. Important clinical symptoms of IgE-mediated allergy include runny nose, itching, drainage, hives (urticaria), eczema, face swelling, vomiting, diarrhoea, noisy breathing or wheeze and are manifested in diseases like allergic asthma, hay fever, allergic rhinitis, allergic eczema, and allergic gastro-intestinal disorders. The allergy is specific in the sense that an individual is sensitized to particular allergen(s) of an allergen source, whereas the individual does not necessarily show an allergic reaction to other allergens from the same allergen source or to allergens from another kind of allergen source associated with IgE-mediated allergy.

The term "slowly administration or slow administration of an allergen composition" is herein meant to denote that the allergen composition is administered to subcutaneous tissue with a significant lower flow rate, such as at least 5 to 10 times lower than usually applied with conventional subcutaneous bolus injection, where an amount of 0.1 to 0.5 ml of allergen composition is typically administered over few seconds to maximally 30 seconds. This conventional applied flow rate corresponds roughly to a flow rate of about 0.4 ml to 1 ml/min. It should be understood that slow administration of the allergen composition to subcutaneous tissue does not, in the present context, includes transdermal or transcutaneous formulations of the allergen composition.

The term "slowly delivery or slow delivery" is herein meant to include that the allergen composition is delivered to subcutaneous tissue with a significant lower flow rate, such as at least 5 to 10 times lower than usually applied with conventional subcutaneous bolus injection, where an amount of 0.1 to 0.5 ml of allergen composition is typically delivered over few seconds to maximally 30 seconds. This conventional applied flow rate corresponds roughly to a flow rate of about 0.4 ml to 1 ml/min. It should be understood that slow delivery of the allergen composition to subcutaneous tissue does not, in the present context, includes transdermal or transcutaneous formulations of the allergen composition.

The terms "administration of allergen composition" and "delivery of allergen composition" are in the present context meant to, to some extent, be interchangeable terms. The term "administration" usually adhere to all kind of situations, where the allergen composition is meant to directly reach the site of administration (here subcutaneous tissue) and where the administration is mainly conducted by a person, a person and a device in common, or by an independently operated device. The term "delivery" in the present context, merely adhere to the specific situations, where an independently operated device (no need for personal supervision) makes it possible for the allergen composition to directly reach the site of administration (here subcutaneous tissue).

The term "subcutaneous infusion" is in the present context meant to designate the administration/delivery of the allergen composition, in fluid form, directly into subcutaneous tissues, usually this through an electronic delivery device, in order to provide a delivery of allergen composition over a substantial longer period than used with subcutaneous injection of the allergen composition in conventional SCIT. The flow is preferably continuous.

The term" infusion pump" is meant to designate a device that is used to deliver quantities of drugs over longer periods of time. They are also commonly called Syringe Pumps, but may include equivalent devices that also are suitable for delivering quantities of drugs to subcutaneous tissue over a period of time, such as a patch pump device (described in the international patent application WO 09015389).

The term "individual in need thereof" refers to any human or other mammals including for example farm animals such as cattle, sheep, pigs, goats and horses, domestic mammals such as dogs and cats. The term does not denote a particular age. Thus, both adult and newborn individuals are intended to be covered. It should be understood that the individual in need thereof belong to a human or other mammal that has symptoms on IgE-mediated allergy or has been found sensitized towards an allergen usually causing IgE-mediated allergy by detecting specific IgE antibodies towards this allergen in their serum or has a positive skin prick test towards an allergen.

The term "kit in part" refers to any set or collection of articles for a specific purpose, here to immunize a human or animal. In the present context, it refers to a packaged set of materials including vials of allergen composition and a device for use in the slow administration/delivery of allergen composition to subcutaneous tissue and optionally instructions or directions. It may be in one or more parts with the parts of the kit in part divided into discrete areas of the package. There may be one or more packages that contain or make up any particular kit in part.

The term, "adjuvant" refers to a substance that enhances the immune response to an antigen. Depending on the nature of the adjuvant it can promote either cell-mediated immune response, humoral immune response or a mixture of the two.

The term "preventing" means any type of prophylactic treatment.

The term "allergen" in the present context is meant to include any proteineaous compound capable of eliciting an IgE-mediated allergic immune response.

As mentioned, the main advantage of this invention relates to the low incidence in immediate and delayed adverse reactions associated with administering an allergen composition to subcutaneous tissue in SCIT treatment, in particularly during the up-dosing phase of SCIT. As shown herein this effect is achieved by subcutaneous infusion of the allergen composition, implying that the allergen composition is continuously delivered to subcutaneous tissue, such as by use of an infusion pump for continuously delivering the allergen composition slowly to subcutaneous tissue.

Therefore, the invention provides in a first aspect an allergen composition for use as defined in the claims.

As mentioned, the slow administration of the allergen is at least conducted in the up-dosing phase, but may also be applied in the maintenance phase if desirable.

Otherwise, the administration of allergen is conducted by subcutaneous bolus injection in the maintenance phase.

It is considered that the subcutaneous infusion or the infusion pump should not be applied with a flow rate exceeding 0.05 ml/min, such as exceeding 0.02 ml/min, 0.015 ml/min or even 0.01 ml/min. Under practical circumstances, it may not be possible to apply a flow rate lower than about 0.0002 ml/min, however, where possible, there is no hindrance for use of an even lower flow rate.

Therefore, in praxis the allergen composition is administered/delivered to subcutaneous tissue of an individual in need thereof by use of subcutaneous infusion, such as by an infusion pump, applied at a flow rate in the range of 0.0002 ml/min to 0.05 ml/min, such as in the range of 0.0005 ml/min to 0.05 ml/min. Typical flow rates will be in the range of 0.002 to 0.05 ml/min.

For example, such flow rates may be accomplished by administering/delivering the following volumes of allergen within the specified period of time;
- 0.1 to 0.5 ml of allergen composition administered/delivered within a period of 10 to 60 minutes (which applies to a flow rate in the range of 0.002 to 0.05 ml/min);
- 0.3 to 0.8 ml of allergen composition administered/delivered within a period of 15 to 90 minutes (which applies to a flow rate in the range of 0.002 to 0.05 ml/min);
- 0.5 to 1.5 ml of allergen composition administered/delivered within a period of 30 to 90 minutes (which applies to a flow rate in the range of 0.006 to 0.05 ml/min); or
- 1 to 2.5 ml of allergen composition administered/delivered within a period of 45 to 180 minutes (which applies to a flow rate in the range of 0.008 to 0.05 ml/min).

Most preferably, the allergen composition is administered/ delivered continuously to subcutaneous tissue which is meant to include that the allergen composition is delivered to subcutaneous tissue during the entire period of administration/delivery with no intention to interrupt the delivery during this period. However, smaller interruptions may occur due to the design of the pump, such as due to natural fluctuation in flow due to pulses. However, where found more suitable, the allergen composition may also be administered discontinuously, which is meant to include that the allergen composition is administered to subcutaneous tissue with one or more periods of non-administration/non-delivery during the entire period of administration/delivery. Preferably, the period of non-administration is shorter than the period of administration. Preferably, the flow rate is kept constant during the continuous administration/delivery, but smaller fluctuations in the flow rate will be acceptable.

Therefore, where smaller fluctuations in the flow rate is desirable or avoidable the flow rate should not be applied with a mean flow rate exceeding 0.05 ml/min, such as exceeding a mean flow rate of 0.02 ml/min, 0.015 ml/min or even 0.01 ml/min. Under practical circumstances, it may not be possible to apply a mean flow rate lower than about 0.0002 ml/min, however, where possible, there is no hindrance for use of an even lower flow rate.

Likewise, under practical circumstances, the allergen composition is administered/delivered to subcutaneous tissue of an individual in need thereof by use of subcutaneous infusion, such as by an infusion pump, applied at a mean flow rate in the range of 0.0002 ml/min to 0.05 ml/min, such as in the range of 0.0005 ml/min to 0.05 ml/min. Typical mean flow rates will be in the range of 0.002 to 0.05 ml/min.

The dose of allergen composition to be administered will be determined by the clinician in accordance with the practice currently applied with SCIT. To some extent, there are at least with respect to allergen extracts, a problem with exploring doses for immunotherapy because there is no international accepted standardisation method. A number of different units of extract strength i.e. bio-potency exist. The methods employed and the units used normally measure the allergen content and biological activity. Examples hereof are SQ-Units (Standardised Quality units), BAU (Biological Allergen Units), BU (biological units), UM (Units of Mass), IU (International Units) and IR (Index of Reactivity). Hence, if extracts of origins other than those disclosed herein are used, they need to be standardised against extract disclosed herein in order to determine their potency in SQ units or any of the above mentioned units. The subject matter is dealt with in "Allergenic extracts", H. Ipsen et al, 1993 and Lowenstein H, 1980.

The bio-potency, i.e. the in vivo allergenic activity, of a given extract depends on a number of factors, the most important being the content of major allergens in the extract, which varies with the composition of the biological source material. Therefore, currently the content of major allergen (micrograms) in the allergen composition remains the only international language to use when comparing allergen compositions from different manufacturers. Suggestions for workable doses for various allergens can be found in table 1 in Nelson H.S, 2007.

Typically, the amount of allergen composition to be administered/ delivered to subcutaneous tissue is in the range of 0.1 ml to 2.5 ml, although higher volumes are not contradicted. It may be understood that where relevant, the allergen composition shall be diluted with a suitable diluents before being administered for which reason higher volumes is delivered to subcutaneous tissue, such as 5 ml or even higher, such as 10 or 20 ml.

Therefore, in interesting embodiments of the invention, an amount of allergen composition in the range of 0.1 ml to 10 ml, such as 0.1 ml to 5 ml, such as 0.1 ml to 2.5 ml is to be administered/ delivered to subcutaneous tissue, usually within a period of no longer than 180 minutes, such as no longer than 90 minutes, such as no longer than 75 minutes, such as no longer than 60 minutes.

For example, an amount of 0.2 ml of the allergen composition may be administered/ delivered within a period of 5 to 30 min (flow rate or mean flow rate is in the range of about 0.013 to 0.007 ml/min); an amount of 0.5 ml of the allergen composition may be administered/ delivered within a period of 15 to 60 min (flow rate or mean flow rate is in the range of about 0.011 to 0.008 min ml/min); an amount of 1.0 ml of the allergen composition may be administered/ delivered within a period of 20 to 90 min (flow rate or mean flow rate is in the range of 0.05 to 0.011 ml/min); an amount of 1.5 ml of the allergen composition may be administered/ delivered within a period of 30 to 90 min (flow rate or mean flow rate is in the range of 0.017 to 0.005 ml/ min) an amount of 2.0 ml of the allergen composition may be administered/ delivered within a period of 45 to 90 min (flow rate or mean flow rate is in the range of 0.022 to 0.044 ml/min) or an amount of 4.0 ml of the allergen composition may be administered/ delivered within a period of 90 to 180 min (flow rate or mean flow rate is in the range of 0.022 to 0.044 ml/min).

It follows that the duration of the administration/delivery can be kept within a period in the range of 5 to 180 minutes, such as preferably in the range of 10 to 180 minutes, such as 10 to 90 minutes, more preferably in the range of 15 to 90 minutes, even more preferably in the range of 15 to 70 minutes. As the slow delivery of an allergen composition has made it possible to reduce the length of the up-dosing phase in SCIT, while still maintaining a good safety profile it is in some embodiments of the invention considered important to administer/deliver the allergen composition to subcutaneous tissue over an even longer period, such as within a period in the range of 20 to 180 minutes or even more preferably in the range of 30 to 180 minutes. Typically, the administration/delivery is within a period in the range of 20 to 90 minutes, such as more typically in the range of 30 to 90 minutes, such as even more typically in the range of 30 to 60 minutes.

The slow delivery/administration of an allergen composition to subcutaneous tissue may be accomplished by any means suitable for this purpose. In one embodiment, the amount of allergen composition is delivered to subcutaneous tissue by means of an infusion pump, such as particularly an infusion pump designed for delivering an amount of the allergen composition within a period in the range of 5 to 180 minutes and/or designed for delivering an allergen composition to subcutaneous tissue with a flow rate in the range of 0.002 ml/min to 0.05 ml/min, such as of 0.002 ml/min to 0.02 ml/min, or more specifically with a mean flow rate in the range of 0.002 ml/min to 0.05 ml/min, such as of 0.002 ml/min to 0.02 ml/min.

It follows that the invention, in enables a kit in part comprising one or more vials, each comprising an allergen composition suitable formulated for subcutaneous administration and an infusion pump or any other device suitable designed for delivering a sufficient amount of allergen composition to subcutaneous tissue by a flow rate in the range from 0.002 ml/min to 0.05 ml/min may be applicable, such as one designed for delivering an amount in the range from 0.1 ml to 5 ml of allergen composition by a flow rate in the range from 0.002 ml/min to 0.05 ml/min. As the present invention has made it possible to use fewer different doses of the allergen composition, it follows that there is only required about maximally 4 different vials with different doses of the allergen composition for completing the up-dosing phase. Preferably, only 2-3 vials are required. A kit in part may be used in SCIT.

Typical examples on infusions pump suitable for this purpose are those allowing for pre-selecting parameters like at least flow rate, volume to be delivered and/or duration of delivery. Within these classes, some pumps are designed to be portable; others are designed to be used in a hospital. For example, an infusion pump for use in this invention is, but not limited to, a portable syringe pump, like a small-volume portable syringe pump. The infusion pump systems that are currently known have included varying degrees of programmability and/or automation. Examples of infusion pump systems that are programmable and/or have some degree of automation include, but are not limited to, those described in United States Patent Nos. 4,670,007 (Wheeldon et al.); 4,978,335(Arthur, III); 4,976,151 (Morshita); 4,856,339 (Williams); 5,256,157 (Samiotes, et al.); 5,756,327 (Sasanfar, et al.); 5,683,367 (Jordan, et al.); 6,269,340 (Ford, et al.); 6,854,620 (Rambetay) and 6,659,980 (Moberg, et al.) as well as United States Patent Application Publication Nos. 2004/0019607 (Moubayed et al.) and 2004/0172283 (Vanderveen et al.).

More suitable, the infusion pump may be disposable, such as the patch pump device described in the international patent application WO09015389.

The infusion pump operated in a continuous manner, where usually small pulses of infusion, depending on the pump's design, with the rate of these pulses depending on the programmed infusion flow rate. Alternatively, the infusion pump may be operated in the intermittent infusion mode, with periods of delivering allergen composition alternating with periods of non-delivery of allergen composition, or periods of delivering allergen composition with high flow rate alternating with a low delivery flow rate. The timings are programmable.

As mentioned, the present invention has made it possible to use fewer doses and administrations of single doses in the up-dosing phase, for which reason the duration of up-dosing phase is decreased significantly. Notably, the number of doses usually applied in conventional SCIT has been reduced from about 7 to 15 administrations of single doses to about 2 to 4 administrations of single doses, depending on the formulation of the allergen composition. Thus, advantageously, the duration of the up-dosing phase is reduced from about 7 to 16 weeks to below one month. Furthermore, it was shown that the significant shortening of the up-dosing phase could be accomplished for various pharmaceutical formulated allergen compositions. Therefore, the present invention has made it possible to generally reduce the duration of the up-dosing phase to about 2 to 4 weeks, independent on the nature of the allergen composition.

As well-known, SCIT treatment is conducted according to particular treatment schedules in respect of doses, number of doses in the up-dosing phase, number of doses in the maintenance phase, start of treatment (e.g. out-side or in-side the pollen season), frequency of dosing in the up-dosing phase (e.g. intervals between administrations), frequency of dosing in the maintenance-phase (e.g. intervals between administrations), and duration of treatment, such as duration of up-dosing phase and maintenance phase.

Typically, the administration by subcutaneous infusion allows for a shorter duration of up-dosing phase, such as duration of 2 to 6 weeks, more typically of 2 to 4 weeks or even more typically of 2 to 3 weeks. The frequency of administering an amount of allergen composition in the up-dosing phase is typically once every two weeks, once a week (weekly), but may also be more frequent, such as daily, such as once daily; every second day; or every third day. In the maintenance phase, the frequency of administering an amount of allergen is typically monthly, such as every fourth week, but longer periods between administrations of allergen may be applied, such as 6 weeks, 8 weeks, 10 weeks or even 12 weeks between administrations.

As mentioned, the number of administrations single doses of the allergen composition in the up-dosing phase is from 1 to 4 administrations, more suitable from 2-3 administrations with typically weekly intervals between the administrations.

Typically, the duration of the maintenance phase is from 12 months to 48 months, preferably from 24 months to 42 months, more preferably from 30 months to 40 months, and most preferably from 34 months to 38 months.

Where SCIT is for treating IgE-mediated allergy caused by seasonal allergens, such as pollen allergens, it is the usual practice to initiate the up-dosing phase long before start of the allergen season, such as at least 10 weeks before. However, due to the improved safety profile provided by the present invention, it is expected that the up-dosing phase may be initiated just before the allergen season and extends into the allergen season or that the up-dosing phase may be initiated at the start of the allergen season or even after the start of the season.

Typical examples on treatment schedules for use in SCIT are, but not limited to;

Schedule 1 comprising an up-dosing phase followed by a maintenance phase, where the up-dosing phase include a single administration of an allergen composition to subcutaneous tissue at a mean flow rate in the range of 0.002 to 0.05 for a period of 15 to 60 minutes at week 0 (start of up-dosing phase), week 1 and week 3 and the maintenance phase include the single administration of an allergen composition by subcutaneous injection with a frequency of 4 weeks between administrations.

Schedule 2 comprising an up-dosing phase followed by a maintenance phase, where the up-dosing phase include a single administration of an allergen composition to subcutaneous tissue at a mean flow rate in the range of 0.002 to 0.05 for a period of 15 to 60 minutes at week 0 week 0 (start of up-dosing phase), week 1, week 2 and week 4 and the maintenance phase include the single administration of an allergen composition by subcutaneous injection with a frequency of 4 weeks between administrations.

### Allergens of the Allergen Composition

The allergen comprises an inhalation allergen associated with IgE-mediated allergy or venom allergens associated with IgE-mediated allergy.

Typical examples of inhalation allergens are pollen allergens from trees, herbs, weed and grass, such as specifically allergens of ragweed, birch, cedar, or grass pollen. Important pollen allergens from trees, grasses and herbs are those originating from the taxonomic orders of Fagales, Oleales, Pinales and platanaceae including i.a. birch (Betula), alder (Alnus), hazel (Corylus), hornbeam (Carpinus) and olive (Olea), cedar (Cryptomeria and Juniperus), Plane tree (Platanus), the order of Poales including i.a. grasses of the genera Lolium, Phleum, Poa, Cynodon, Dactylis, Holcus, Phalaris, Secale, and Sorghum, the orders of Asterales and Urticales including i.a. herbs of the genera Ambrosia, Artemisia, and Parietaria.

Further typical examples of inhalation allergens are those from house dust mites of the genus Dermatophagoides and Euroglyphus, storage mite e.g Lepidoglyphys, Glycyphagus, Tyrophagus and from the genus Blomia (e.g. Blomia tropicalis and/or Blomia freemani), those from cockroaches, midges and fleas e.g. Blatella, Periplaneta, Chironomus and Ctenocepphalides.

Still further typical examples of inhalation allergens are animal dander and hair allergens from cat, dog, horse, rat and mice. Other examples of inhalation allergens are from fungi i.a. such originating from the genera Alternaria and Cladosporium.

Typical examples of insect allergens are insect venom allergens, e.g hymenopthera venom allergens. Venom allergens include such originating from stinging or biting insects such as those from the taxonomic order of Hymenoptera including bees (superfamily Apidae), wasps (superfamily Vespidea), and ants (superfamily Formicoidae).

In a particular embodiment of the invention the allergen or a combination of allergens may be selected from the following list of allergens: when the allergen is an inhalation allergen, said inhalation allergen is selected from the group consisting of
- a pollen allergen from a tree, such as a tree selected from the group consisting of Betula, Alnus, Corylus, Carpinus, Olea, Cryptomeria, Juniperus, and Platanus,
- a pollen allergen from a herb, such as a herb selected from the group consisting of Ambrosia, Artemisia, and Parietaria,
- a pollen allergen from a weed,
- a pollen allergen from a grass, such as a grass selected from the group consisting of Lolium, Phleum, Poa, Cynodon, Dactylis, Holcus, Phalaris, Secale, and Sorghum,
- an animal dander allergen from a cat, a dog, or a horse,
- a house dust mite of the genus Dermatophagoides, a house dust mite of the genus Euroglyphus and a storage mite of the genus Blomia, and
- a fungus,
and when the allergen is a venom allegen, said venom allergen is a hymenopthera allergen.

In presently preferred embodiments of the invention the allergen is birch pollen allergen, cat dander or hair allergen, cedar pollen allergen, dust mite allergen, grass pollen allergen, hymenopthera venom allergen or ragweed pollen allergen.

The allergen composition may comprise an extract of natural allergen sources like pollen, dander, hair or venoms, a purified allergen, a modified allergen, a recombinant allergen or a mutant of a recombinant allergen or mixtures thereof. An extract may naturally contain one or more iso-forms of the same allergen, whereas a recombinant allergen typically only represents one iso-form of an allergen. The allergen composition may comprise low IgE binding allergens according to WO 99/47680, WO 02/40676 or WO 03/096869 A2.

### Formulation of the Allergen Composition

The allergen composition according to the present invention may be any formulation suitable for being administered by the new manner of subcutaneous administration. Typically, this includes a liquid including a solution, a suspension, a dispersion, a gelled liquid. Alternatively, the allergen composition is an emulsion or a re-dissolvable powder, granulate or lyophilisate, which can be dissolved to form a liquid before being administered.

The allergen composition may further comprise any adjuvant and other excipients suitable for such type of formulation. Such excipients are well-known to the person skilled in the art and include solvents, emulsifiers, wetting agents, plasticizers, colouring substances, fillers, preservatives, viscosity adjusting agents, buffering agents, pH adjusting agents, isotonicity adjusting agents, mucoadhesive substances, and the like. Examples of formulation strategies are well-known to the person skilled in the art.

The adjuvant may be any conventional adjuvant, including oxygen-containing metal salts, e.g. aluminium hydroxide, heat-labile enterotoxin (LT), cholera toxin (CT), cholera toxin B subunit (CTB), polymerised liposomes, mutant toxins, e.g. LTK63 and LTR72, microcapsules, interleukins (e.g. IL-1 BETA, IL-2, IL-7, IL-12, INFGAMMA), GM-CSF, MDF derivatives, CpG oligonucleotides, LPS, MPL, phosphophazenes, Adju-Phos(R), glucan, antigen formulation, liposomes, DDE, DHEA, DMPC, DMPG, DOC/Alum Complex, Freund's incomplete adjuvant, ISCOMs(R), LT Oral Adjuvant, muramyl dipeptide, monophosphoryl lipid A, muramyl thpeptide, and phospatidylethanolamine.

According to a particular embodiment of the invention, the adjuvant is an oxygen-containing metal salts, e.g. aluminium hydroxide, which typically forms a gel. The concentration of aluminium hydroxide in the formulation is preferably 0.035-1000 mg/ml, more preferably 0.10-100 mg/ml, more preferably 0.25-10 mg/ml, and most preferably 0.5-5 mg/ml. However, the formulation of the invention may also have the form of a highly concentrated gel or gel-like formulation.

It is believed that the adjuvant activity of aluminium hydroxide include the activation of inflammasome, where basically a massive danger signal induced by local tissue damage caused by the aluminium hydroxide in the injection site causes activation of the immunological system (Ennio De Gregorio *et al* 2008).

Preferably, the metal cation of the oxygen-containing metal salt is selected from the group consisting of Al, K, Ca, Mg, Zn, Ba, Na, Li, B, Be, Fe, Si, Co, Cu, Ni, Ag, Au and Cr, preferably aluminium.

The anion of the oxygen-containing compound may be any oxygen- containing anion, including an organic or inorganic anion, or a combination of organic and inorganic anions. Examples of suitable oxygen-containing metal salts are e.g. those, wherein the anion is selected from the group consisting of sulphates, hydroxides, phosphates, nitrates, iodates, bromates, carbonates, hydrates, acetates, citrates, oxalates, and tartrates, as well as mixed forms thereof. The oxygen-containing metal salts further comprise coordination complexes. A definition of coordination complexes is given in e.g. The Handbook of Chemistry and Physics 56 Ed., Section B, Chapter 7 (1975).

Within the present context, the expression "mixed forms" is intended to include combinations of the various anions as well as combinations with e. g. chlorides, and sulphides.

Examples of oxygen-containing metal salts according to the invention are aluminium hydroxide, aluminium phosphate, aluminium sulphate, aluminium acetate, potassium aluminium sulphate, calcium phosphate, calcium tartrate, Maalox (mixture of aluminium hydroxide and magnesium hydroxide), beryllium hydroxide, zinc hydroxide, zinc carbonate, zinc sulphate, and barium sulphate.

Most preferred are aluminium hydroxide, aluminium phosphate, aluminium acetate, calcium phosphate, calcium tartrate and zinc sulphate.

The pi of the oxygen-containing metal salt is typically in the range of 2-11. The pi for allergen proteins is typically in the range of 4-9. Preferably, the allergen and oxygen-containing metal salt are selected so that the pi of the allergen is lower than the pi of the oxygen-containing metal salt.

When using e.g. aluminium hydroxide as oxygen-containing metal salt, the concentration of aluminium hydroxide in the formulation is preferably 0.035- 1000 mg/ml, more preferably 0.10-100 mg/ml, more preferably 0.25-10 mg/ml, and most preferably 0.5-5 mg/ml. For the other oxygen-containing metal salts, the concentration of the metal salt is preferably 0.035-1000 mg/ml, more preferably 0.35-100 mg/ml, more preferably 0.7-50 mg/ml, and most preferably 1.0-20 mg/ml. The concentration of allergen in the formulation is preferably 0.01-100 mg/ml, more preferably 0.1-10 mg/ml. The ratio of oxygen-containing metal salt to allergen is preferably from 0.1 to 100, more preferably from 1 to 20. The degree of allergen adsorbed to the oxygen-containing metal salt is typically from 5 to 99 %, more preferably from 10 to 99 % of the added amount. The adsorption of allergen to the oxygen- containing metal salt depends on the buffer system and the reaction conditions, including temperature and reaction time, under which the adsorption takes place.

It is believed that the biologically active substance is adsorbed (or coupled) to the oxygen-containing metal salt, and this adsorption contributes to the efficacy of the vaccine. Several factors may be important or influence the adsorption between the active substance and the oxygen-containing metal salt (see e. g. P. M. Callahan *et al.,* 1991). These factors include pH, the length of time the adsorption reaction is carried out for, mixing conditions, concentrations of the various components in the vaccines, containers, temperature, storage, buffer and excipients. It has further been found that the adsorption of the active substance may be influenced by the net/overall charge of the metal salt and the charge of the active substance, both of which are pH dependent. A further feature believed to be of importance is the solubility of the oxygen- containing metal salts.

### List of References

Bousquet J, Lockey R, Malling HJ, Alvarez-Cuesta E. Allergen immunotherapy: therapeutic vaccines for allergic diseases. World Health Organization. American academy of Allergy, Asthma and Immunology. Allergy;53(44):1-42, 1998.
Callahan PM et al., Vaccine Design. The Subunit and Adjuvant Approach, Pharmaceutical Research Vol. 8, No. 7, 851-858, 1991.
Casanovas M, Martin R, Jiménez C, Caballero R, Fernández-Caldas E. Safety of an ultra-rush immunotherapy build-up schedule with therapeutic vaccines containing depigmented and polymerized allergen extracts. Int Arch Allergy Immunol.139(2):153-8, 2006.
Castera V, Dutour-Meyer A, Koeppel MC, Petisjean C, Darmon P; systemic allergy to human insulin and its rapid and long acting analogs: succesfull treatment by continuous subcutaneous subcutaneous lispro infusion. Diabetes Metab 2005; 31; 391-400.
Cox L. Advantages and disadvantages of accelerated immunotherapy schedules. J Allergy Clin Immunol. 122(2):432-4, 2008.
Gardulf A, Andersson E, Lindquist M, Hansen S, Gustafson R. Rapid subcutaneous IgG replacement therapy at home for pregnant immunodeficient women. Journal of Clinical Immunology;21(2):150-154, 2001.
Gregorio ED, Tritto E Rappuoli R. Alum adjuvanticity: Unraveling a century old mystery. (Eur. J. Immunol. 38: 2068-2071, 2008.
Ipsen H et al, chapter 20 in Allergy, principle and practise (Ed. S. Manning) 1993, Mosby-Year Book, St. Louis.
Kinchi MS, Poulsen LK. Clinical efficacy of sublingual and subcutaneous birch pollen allergen specific immunotherapy: a randomized, placebo-controlled, double-blind, double-dummy study. Allergy 59; 45-53, 2004.
Lowenstein H. Arb Paul Ehrlich Inst 75:122, 1980.
Moyes V, Driver R, Mirikian R, Chowdhury T: Insulin allergy in a patient with type 2 diabetes treated continuous subcutaneous insulin infusion, Clinical and Experimental allergy, 35; 1410-1418, abstract p 1417, 2005.
Nagai T, Nagai Y, Tomizawa T and Mori M.Immediate-type human insulin allergy successfully treated by continuous subcutaneous insulin infusion. Internal Medicine, 36, 575-578, 1997.
Nelson H.S. Allergen immunotherapy: where is it now?, J Allergy Clim Immunol, p 769-771, 2007.
Nouri-Aria KT, Durham SR. Regulatory T cells and allergic disease. Inflamm Allergy Drug Targets, 7(4), 237-52, 2008.
Quirino, T; Lemoli, E. Sublingual versus injective immunotherapy in grass pollen allergic patients: a double blind (double dummy) study Clinical & Experimental Allergy, Vol 26, No 11, pp. 1253-1261, 1996.

### Examples

### Example 1

The invention is exemplified by the following experiments that surprisingly demonstrate that the incidence in adverse reactions, like immediate and delayed reactions, is not enhanced by reducing the duration of the up-dosing phase and by reducing the number of doses required to achieve the maintenance dose in comparison to conventional SCIT

According to a clinical protocol, a number of patients allergic to at least one allergen selected from different species, such as pollen (grass, grass + olive tree, cypress tree), venoms (bee or wasp) hair and dander (cat, dog) and fungi (alternaria) are included in this experiment.

Various formulations of allergen compositions (commercialised under the names Alutard®, Pangramin® and Pharmalgen®) comprising at least one allergen selected from different species, such as pollen (grass, grass + olive tree, cypress tree), venoms (bee or wasp) hair and dander (cat, dog) and fungi (alternaria) is used.

A pre-selected amount of allergen composition is administered to subcutaneous tissue to the patient via an infusion pump (here INFUSA^{®}T1) that deliver the allergen composition in a programmed time, for example 30 minutes to deliver 0.5 ml allergen composition and 45 minutes to deliver 1 ml of allergen composition. After administration, the patients remain under medical supervision for 30 minutes. The specific treatment schedules are detailed below. After reaching the established maintenance dose, the administration for each allergen composition is switched to the standard one, namely standard subcutaneous injection without the use of infusion pump. If an adverse reaction arises at any dose, it will be recorded and collected.

The following treatment schedules were used:

| Schedule 1 | | | | |
|---|---|---|---|---|
| Week | Phase | Amount of Allergen Composition (ml) | % Dose of Maintenance Dose | Manner of Administration |
| 0 | Up-dosing | 0.5 | 62,5 | Infusion pump (30 min) |
| 1 | Up-dosing | 1 | 125 | Infusion pump (45 min) |
| 3 | Up-dosing | 1 | 125 | Infusion pump (45 min) |
| 7 | Maintenance | 0.8 | 100 | WITHOUT infusion pump |
| 11 | Maintenance | 0.8 | 100 | WITHOUT infusion pump |

| Schedule 2 | | | | |
|---|---|---|---|---|
| Week | Phase | Amount of Allergen Composition (ml) | % Dose of Maintenance Dose | Manner of Administration |
| 0 | Up-dosing | 1 | 10 | Infusion pump (45 min) |
| 1 | Up-dosing | 0.5 | 50 | Infusion pump (30 min) |
| 2 | Up-dosing | 1 | 100 | Infusion pump (45 min) |
| 4 | Up-dosing | 1 | 100 | Infusion pump (45 min) |
| 8 | Maintenance | 1 | 100 | WITHOUT infusion pump |
| 12 | Maintenance | 1 | 100 | WITHOUT infusion pump |

| Schedule 3 | | | | |
|---|---|---|---|---|
| Week | Phase | Amount of Allergen Composition (ml) | % Dose of Maintenance Dose | Manner of Administration |
| 0 | Up-dosing | 0,5 | 5 | Infusion pump (30 min) |
| 1 | Up-dosing | 0,5 | 50 | Infusion pump (30 min) |
| 2 | Up-dosing | 1 | 100 | Infusion pump (45 min) |
| 4 | Up-dosing | 1 | 100 | Infusion pump (45 min) |
| 8 | Maintenance | 1 | 100 | WITHOUT infusion pump |
| 12 | Maintenance | 1 | 100 | WITHOUT infusion pump |

### Results

It was found that for the various allergen compositions tested, it was possible to reduce the number of administrations in the up-dosing phase; it was possible to significantly shorten standard treatment schedules to reach the maintenance dose, while keeping at least the same safety profile as the standard treatment has. It was further demonstrated that it was possible to switch from administering by infusion pump to direct subcutaneous injection without any safety concern.

The following dose numbers were found to be required to reach the established maintenance dose by use of the new manner of administering an allergen composition:

| Allergen Composition | Dose no. needed to reach the established maintenance dose in conventional treatment | Dose no. needed to reach the established maintenance dose with the *infusion pump* |
|---|---|---|
| Alutard^{®} | 15 | 3 |
| Pangramin^{®} Plus | 7 | 2 |
| Pangramin^{®} Depot | 13 | 2 |
| Pharmalgen^{®} | 12 | 3 |

## Claims

1. An allergen composition comprising an inhalation allergen or a venom allergen associated with IgE-mediated allergy, formulated to be suitable for subcutaneous administration, for use in subcutaneous specific allergen immunotherapy, which comprises an up-dosing phase and a maintenance phase, and a dose of the allergen composition in the up-dosing phase is administered to an individual in need thereof by use of subcutaneous infusion using a continuous flow at a flow rate not exceeding 0.05 ml/min within a period in the range of 5 to 180 minutes and wherein the up-dosing has a duration of 2-6 weeks and the number of administrations of single doses in the up-dosing phase is from 2-4.

2. The allergen composition for the use according to claim 1, wherein the subcutaneous infusion is provided by an infusion pump operated at a flow rate not exceeding 0.05 ml/min.

3. The allergen composition for the use according to any one of the preceding claims, wherein the frequency of administering the allergen composition in the up-dosing phase is once every two weeks or once a week.

4. The allergen composition for the use according to any one of the preceding claims, wherein the allergen composition is administered to subcutaneous tissue within a period in the range of 10 to 90 minutes.

5. The allergen composition for the use according to any one of the preceding claims, wherein the allergen composition is administered to subcutaneous tissue within a period in the range of 15 to 70 minutes.

6. The allergen composition for the use according to any one of the preceding claims, wherein 0.1 to 0.5 ml of the allergen composition is administered within a period of 10 to 60 minutes.

7. The allergen composition for the use according to any one of the preceding claims, wherein 0.3 to 0.8 ml of the allergen composition is administered within a period of 15 to 90 minutes.

8. The allergen composition for the use according to any one of the preceding claims, wherein 0.5 to 1.5 ml of the allergen composition is administered within a period of 30 to 90 minutes.

9. The allergen composition for the use according to any one of the preceding claims, wherein, when the allergen is an inhalation allergen, said inhalation allergen is selected from the group consisting of
- a pollen allergen from a tree, such as a tree selected from the group consisting of Betula, Alnus, Corylus, Carpinus, Olea, Cryptomeria, Juniperus, and Platanus,
- a pollen allergen from a herb, such as a herb selected from the group consisting of Ambrosia, Artemisia, and Parietaria,
- a pollen allergen from a weed,
- a pollen allergen from a grass, such as a grass selected from the group consisting of Lolium, Phleum, Poa, Cynodon, Dactylis, Holcus, Phalaris, Secale, and Sorghum,
- an animal dander allergen from a cat, a dog, or a horse,
- a house dust mite of the genus Dermatophagoides, a house dust mite of the genus Euroglyphus and a storage mite of the genus Blomia, and
- a fungus,
and wherein, when the allergen is a venom allegen, said venom allergen is a hymenopthera allergen.

10. The allergen composition for use in subcutaneous specific allergen immunotherapy according to any one of claims 1 to 9, wherein the allergen composition comprises an extract of a natural allergen, a purified allergen, a modified allergen or a recombinant allergen.

## Patentansprüche

1. Allergenzusammensetzung, welche ein Inhalationsallergen oder ein Giftallergen im Zusammenhang mit einer IgE-mediierten Allergie umfasst, derart formuliert, dass sie für eine subkutane Verabreichung geeignet ist, zur Verwendung bei einer subkutanen spezifischen Allergenimmuntherapie, welche eine Hochdosierungsphase und eine Erhaltungsphase umfasst, wobei eine Dosis der Allergenzusammensetzung in der Hochdosierungsphase einem Individuum mit Bedarf daran durch Verwendung einer subkutanen Infusion unter Verwendung eines kontinuierlichen Stromes mit einem Durchfluss, der 0,05 ml/min nicht übersteigt, innerhalb eines Zeitraums im Bereich von 5 bis 180 Minuten verabreicht wird und wobei die Hochdosierung eine Dauer von 2-6 Wochen hat und die Zahl der Verabreichungen einzelner Dosen in der Hochdosierungsphase von 2-4 ist.

2. Allergenzusammensetzung zur Verwendung nach Anspruch 1, wobei die subkutane Infusion durch eine Infusionspumpe bereitgestellt wird, die mit einem Durchfluss betrieben wird, der 0,05 ml/min nicht übersteigt.

3. Allergenzusammensetzung zur Verwendung nach einem der vorhergehenden Ansprüche, wobei die Häufigkeit der Verabreichung der Allergenzusammensetzung in der Hochdosierungsphase einmal alle zwei Wochen oder einmal die Woche ist.

4. Allergenzusammensetzung zur Verwendung nach einem der vorhergehenden Ansprüche, wobei die Allergenzusammensetzung innerhalb eines Zeitraums im Bereich von 10 bis 90 Minuten an subkutanes Gewebe verabreicht wird.

5. Allergenzusammensetzung zur Verwendung nach einem der vorhergehenden Ansprüche, wobei die Allergenzusammensetzung innerhalb eines Zeitraums im Bereich von 15 bis 70 Minuten an subkutanes Gewebe verabreicht wird.

6. Allergenzusammensetzung zur Verwendung nach einem der vorhergehenden Ansprüche, wobei 0,1 bis 0,5 ml der Allergenzusammensetzung innerhalb eines Zeitraums von 10 bis 60 Minuten verabreicht werden.

7. Allergenzusammensetzung zur Verwendung nach einem der vorhergehenden Ansprüche, wobei 0,3 bis 0,8 ml der Allergenzusammensetzung innerhalb eines Zeitraums von 15 bis 90 Minuten verabreicht werden.

8. Allergenzusammensetzung zur Verwendung nach einem der vorhergehenden Ansprüche, wobei 0,5 bis 1,5 ml der Allergenzusammensetzung innerhalb eines Zeitraums von 30 bis 90 Minuten verabreicht werden.

9. Allergenzusammensetzung zur Verwendung nach einem der vorhergehenden Ansprüche, wobei, wenn das Allergen ein Inhalationsallergen ist, das Inhalationsallergen ausgewählt ist aus der Gruppe bestehend aus
- einem Pollenallergen von einem Baum, wie z.B. einem Baum ausgewählt aus der Gruppe bestehend aus Betula, Alnus, Corylus, Carpinus, Olea, Cryptomeria, Juniperus und Platanus,
- einem Pollenallergen von einem Kraut, wie z.B. ein Kraut ausgewählt aus der Gruppe bestehend aus Ambrosia, Artemisia und Parietaria,
- einem Pollenallergen von einem Unkraut,
- einem Pollenallergen von einem Gras, wie z.B. ein Gras ausgewählt aus der Gruppe bestehend aus Lolium, Phleum, Poa, Cynodon, Dactylis, Holcus, Phalaris, Secale und Sorghum,
- einem Tierhaarallergen von einer Katze, einem Hund oder einem Pferd,
- einer Hausstaubmilbe der Gattung Dermatophagoides, einer Hausstaubmilbe der Gattung Euroglyphus und einer Vorratsmilbe der Gattung Blomia, und
- einem Pilz,
und wobei, wenn das Allergen ein Giftallergen ist, das Giftallergen ein Hymenoptera-Allergen ist.

10. Allergenzusammensetzung zur Verwendung bei einer subkutanen spezifischen Allergenimmuntherapie nach einem der Ansprüche 1 bis 9, wobei die Allergenzusammensetzung einen Extrakt eines natürlichen Allergens, ein aufgereinigtes Allergen, ein modifiziertes Allergen oder ein rekombinantes Allergen umfasst.

## Revendications

1. Composition d'allergène comprenant un allergène respiratoire ou un allergène de venin associé à une allergie médiée par les IgE, formulée pour être appropriée pour une administration sous-cutanée, pour son utilisation dans une immunothérapie sous-cutanée contre un allergène spécifique, qui comprend une phase d'augmentation de dose et une phase d'entretien, et une dose de la composition d'allergène dans la phase d'augmentation de dose est administrée à un individu le nécessitant par perfusion sous-cutanée en utilisant un flux continu à un débit n'excédant pas 0,05 mL/minute en une période de la plage de 5 à 180 minutes et dans laquelle l'augmentation de dose a une durée de 2 à 6 semaines et le nombre d'administrations de doses uniques dans la phase d'augmentation de dose est de 2 à 4.

2. Composition d'allergène pour son utilisation selon la revendication 1, dans laquelle la perfusion sous-cutanée est fournie par une pompe à perfusion fonctionnant à un débit n'excédant pas 0,05 mL/minute.

3. Composition d'allergène pour son utilisation selon l'une quelconque des revendications précédentes, dans laquelle la fréquence d'administration de la composition d'allergène dans la phase d'augmentation de dose est d'une fois toutes les deux semaines ou d'une fois par semaine.

4. Composition d'allergène pour son utilisation selon l'une quelconque des revendications précédentes, dans laquelle la composition d'allergène est administrée à un tissu sous-cutané en une période dans la plage de 10 à 90 minutes.

5. Composition d'allergène pour son utilisation selon l'une quelconque des revendications précédentes, dans laquelle la composition d'allergène est administrée à un tissu sous-cutané en une période dans la plage de 15 à 70 minutes.

6. Composition d'allergène pour son utilisation selon l'une quelconque des revendications précédentes, dans laquelle de 0,1 à 0,5 mL de la composition d'allergène est administré en une période de 10 à 60 minutes.

7. Composition d'allergène pour son utilisation selon l'une quelconque des revendications précédentes, dans laquelle 0,3 à 0,8 mL de la composition d'allergène est administré en une période de 15 à 90 minutes.

8. Composition d'allergène pour son utilisation selon l'une quelconque des revendications précédentes, dans laquelle 0,5 à 1,5 mL de la composition d'allergène est administré en une période de 30 à 90 minutes.

9. Composition d'allergène pour son utilisation selon l'une quelconque des revendications précédentes, dans laquelle l'allergène est un allergène respiratoire, ledit allergène respiratoire est sélectionné dans le groupe constitué de
- un allergène de pollen d'un arbre, tel qu'un arbre sélectionné dans le groupe constitué du bouleau, de l'aulne, du noisetier, du charme, de l'olivier, du cèdre, du genévrier, et du platane,
- un allergène de pollen d'une herbe, telle qu'une herbe sélectionnée dans le groupe constitué de l'ambroisie, de l'armoise, et de la pariétaire,
- un allergène de pollen d'une mauvaise herbe,
- un allergène de pollen d'une herbe graminiforme, telle qu'une herbe graminiforme sélectionnée dans le groupe constitué du ray-grass, de la fléole, des poacées, du cynodon, du dactyle aggloméré, de la houlque, de la phalaride, du seigle, et du sorgho,
- un allergène de phanères d'animal d'un chat, d'un chien, ou d'un cheval,
- un acarien de la poussière de maison du genre Dermatophagoïdes, un acarien de la poussière de maison du genre Euroglyphus et un acarien de stockage du genre Blomia, et
- un champignon,
et dans laquelle, lorsque l'allergène est un allergène de venin, ledit allergène de venin est un allergène d'hyménoptères.

10. Composition d'allergène pour son utilisation dans une immunothérapie sous-cutanée contre un allergène spécifique selon l'une quelconque des revendications 1 à 9, dans laquelle la composition d'allergène comprend un extrait d'un allergène naturel, d'un allergène purifié, d'un allergène modifié ou d'un allergène recombinant.
